# FASCICULE DE BREVET EUROPEEN

(11) **EP 4 093 193 B1**
(45) Date de publication et mention de la délivrance du brevet: **11.10.2023**
(21) Numéro de dépôt: 21700019.9
(22) Date de dépôt: 06.01.2021
(51) Int. Cl.: A01K 67/033

(54) **SYSTÈME ET PROCÉDÉ DE MANUTENTION DE BACS DE PRODUCTION D'INSECTES**
SYSTEM UND VERFAHREN ZUR HANDHABUNG VON SCHALEN FÜR DIE INSEKTENPRODUKTION
SYSTEM AND METHOD FOR HANDLING INSECT PRODUCTION TRAYS

(30) Priorité: 21.01.2020 FR 2000550
(43) Date de publication de la demande: 30.11.2022
(73) Titulaire: Invers, 63720 Saint-Ignat (FR)
(72) Inventeur: CREPIEUX, Sébastien, 63720 Saint-Ignat (FR); TOURNIER, Ludovic, 63720 Saint-Ignat (FR); QUITTARD, David, 63720 Saint-Ignat (FR); CAILLOUX, Stéphanie, 63720 Saint-Ignat (FR)
(74) Mandataire: Gabriel, Franck
(86) Numéro de dépôt international: PCT/IB2021/050073
(87) Numéro de publication internationale: WO 2021/148894

(56) Documents cités:
- WO-A1-2019/059760
- US-A1- 2019 387 704

## Description

### DOMAINE TECHNIQUE DE L'INVENTION

La présente invention concerne un système et un procédé de manutention de bacs de production d'insectes, pour une installation de production d'insectes, notamment pour l'alimentation animale.

### ETAT DE LA TECHNIQUE ANTERIEURE

Diverses approches existent pour mettre en oeuvre l'élevage d'insectes à grande échelle. Les premières méthodes étaient entièrement manuelles, fastidieuses et avec des taux de productivité peu élevés. Peu à peu, la technicité des équipements utilisés a permis de réduire les interventions humaines dans le processus et d'augmenter la productivité. Aujourd'hui, de nombreux systèmes avec divers équipements technologiques existent.

La production d'insectes à échelle industrielle implique en général deux cycles ou phases complémentaires, soit un premier pour obtenir des oeufs, et un second pour permettre la croissance des larves ainsi obtenues. Ces deux cycles ayant chacun leurs spécificités sont aujourd'hui réalisés de façon totalement indépendante, avec des moyens spécifiques à chacun.

Par exemple, le document WO2016153340 décrit un système et un procédé pour effectuer la reproduction des insectes à l'aide de reproducteurs produisant des oeufs. Le système est particulièrement adapté pour les cycles de reproduction et ne convient pas pour effectuer des cycles de production d'insectes à partir des oeufs. Pour effecteur les deux cycles, une installation industrielle doit donc prévoir deux types de systèmes différents, soit un pour produire les oeufs, et un pour produire les larves et/ou insectes. Une telle installation requière une grande surface, présente une double complexité, et occasionne un surcoût important.

Le document FR3034622 décrit un atelier d'élevage d'insectes, comportant une première zone dans laquelle les insectes en cours d'élevage sont stockés au cours de leur croissance dans des contenants et une seconde zone comportant au moins un poste configuré pour la réalisation d'une opération d'élevage sur les insectes d'un contenant ou sur ledit contenant. Les contenants sont groupés dans la première zone en ensembles de contenants palettisés dits unités élémentaires. La première zone comporte des rayonnages à palettes dans lesquels sont disposées les unités élémentaires. La première zone est en outre équipée d'un dispositif automatisé configuré pour le déplacement des unités élémentaires entre la première zone et une interface avec la seconde zone. L'atelier prévoit, dans la seconde zone, une pluralité de postes spécialisés chacun conçu pour effectuer une opération spécifique. Il en résulte une installation complexe, coûteuse, requérant une très grande surface.
Le document US2019/387704 est considéré comme l'état de la technique le plus proche de l'objet des revendications et décrit un système de récolte d'insectes à partir d'un habitat Le système est configuré pour séparer les déchets, les insectes vivants et les insectes morts de manière à ce que les insectes vivants puissent subir une première procédure de traitement et que les insectes morts puissent subir une seconde procédure de traitement. Par exemple, la première procédure de traitement peut comprendre le traitement des insectes pour la consommation humaine et la seconde procédure de traitement peut comprendre le traitement des insectes morts pour les minéraux ou les matériaux.
Le document WO2019/059760 décrit un système pour l'élevage de larves d'insectes, comprenant une multitude de caisses empilables similaires, un logement climatique et un équipement d'empilage de caisses, dans lequel la multitude de caisses similaires ont chacune une même largeur D1, une même longueur D2, une même structure d'angle qui comprend des éléments inférieurs et des éléments supérieurs complémentaires, configurés pour permettre l'empilage des caisses les unes sur les autres en permettant aux éléments supérieurs d'une caisse inférieure d'interagir avec les éléments inférieurs d'une caisse supérieure, et une zone de confinement définie par la largeur, la longueur et une hauteur de la caisse.

Pour pallier ces différents inconvénients, l'invention prévoit différents moyens techniques.

### EXPOSE DE L'INVENTION

Tout d'abord, un premier objectif de l'invention consiste à fournir un système et un procédé permettant de traiter de façon rapide et fiable des lots de bacs en fin de cycle de production et de préparer les lots suivants pour les prochains cycles de production.

Un autre objectif de l'invention consiste à fournir un système et un procédé uniques pour traiter les deux cycles requis pour effectuer une production industrielle d'insectes, soit un cycle pour produire des oeufs, et un cycle pour produire des larves.

Pour ce faire, l'invention prévoit un système de manutention de bacs de production d'insectes, pour effectuer la transition entre la fin d'un cycle de production d'une pile de bacs de fin de cycle avec transvasement de la matière de production de chacun des bacs de la pile de fin de cycle, la séparation et récupération des constituants de la matière de production, et l'amorce d'un nouveau cycle de production avec remplissage de bacs de matière d'amorce de production et constitution d'une pile de bacs d'amorce de cycle, ledit système comprenant :
i) un portique d'entrée et sortie de piles de bacs pour recevoir et mettre en attente une pile de bacs de fin de cycle à traiter et disposer une pile de bacs d'amorce de cycle;
ii) un poste de transition entre une fin de cycle pour une pile de bacs de fin de cycle et une amorce d'un nouveau cycle pour constituer une pile de bacs d'amorce de cycle, le poste de transition comprenant :
   - un tamiseur-récupérateur pour matière de fin de cycle pour séparer les constituants à récupérer de la matière de fin de cycle de production et acheminer ces constituants vers des points de récupération;
   - une station de remplissage de bacs comprenant une pluralité de doseurs de constituants de matière d'amorce de cycle de production;
iii) un bras manipulateur pour dépiler séparément chaque bac de la pile de bacs de fin de cycle à traiter, acheminer chaque bac vers le tamiseur-récupérateur , verser le contenu de chaque bac dans le tamiseur-récupérateur, poser les bacs vides dans une zone de réception de bacs vides, saisir un bac nouvellement rempli dans la station de remplissage, le transporter vers une pile de bacs d'amorce en cours de constitution et effectuer l'empilage des bacs de cette pile.

L'architecture prévoit un îlot de travail unique et compact pour assurer à la fois les opérations de séparation et de récupération des constituants de la matière de fin de cycle de production et les opérations de remplissage de bacs d'amorce de cycle avec des matières d'amorce de production.

Le poste de transition forme un combiné qui permet de concentrer les opérations de dépilage, acheminement, transvasement, séparation et récupération pour chaque bac d'une pile de bacs de fin de cycle ET les opérations de remplissage, acheminement et empilage pour chaque bac de la pile d'amorce de cycle. L'agencement en série des opérations de séparation et récupération des matières de fin de cycle et des opérations de remplissage des bacs pour de nouveaux cycles de production permet de réaliser une production continue, sans devoir stocker les bacs entre deux cycles de production.

Le portique unique d'entrée et sortie de piles de bacs permet de recevoir une pile de bacs de fin de cycle pour mise en attente, dépilage et traitement de chacun des bacs, et de disposer, à proximité immédiate de la pile de fin de cycle, une pile d'amorce de cycle en cours de constitution. Il en résulte un flux de piles minimaliste, et une gestion de flux simple, et un emplacement réduit.

Cet agencement permet de réaliser l'ensemble des manipulations à l'aide d'un seul et unique outil de manipulation, contribuant à minimiser l'espace requis et à réduire les coûts du système. Cet outil de manipulation est multi-phases afin de réaliser les opérations pour les deux phases en cours, à savoir une phase de récupération de matière de production d'un cycle de production achevé et une phase de préparation de bacs avec ajout de matière d'amorce d'un nouveau cycle de production. On dispose avantageusement le bras manipulateur entre les piles de bacs et le poste de transition.

On utilise avantageusement des bacs de dimensions relativement imposantes, par exemple de 800 cm par 600 cm. Une fois chargés, les bacs sont trop lourds pour pouvoir être manipulés manuellement par un opérateur lors des phases de fonctionnement à échelle industrielle, impliquant de manipuler plusieurs bacs par minute. Le bras manipulateur permet d'effectuer ces opérations sans affecter la sécurité des opérateurs, qui peuvent ainsi se concentrer sur les opérations de contrôle et surveillance.

Selon un mode de réalisation avantageux, le tamiseur-récupérateur comporte au moins deux étages.

Le tamiseur est un organe efficace et performant pour effectuer les opérations de sélection ou tri des constituants du contenu des bacs, même pour des bacs de grande dimension. Le tamiseur permet aisément de traiter tout le contenu d'un bac entier. La superposition des étages, avec des tamis de granulométrie différente, permet d'effectuer sur une surface contenue plusieurs opérations de séparation avec acheminement vers des points de récupération des constituants.

De manière avantageuse, la récupération des constituants pour un cycle de ponte et la récupération des constituants pour un cycle de production de larves ou d'insectes peuvent être effectuée dans le même tamiseur-récupérateur. Le tamiseur-récupérateur multi-cycles permet d'adapter le système de manutention de façon simple et rapide, à des coûts réduits, et dans un emplacement restreint.

Selon un autre mode de réalisation avantageux, le tamiseur-récupérateur comprend un système d'aspiration agencé pour récupérer les mues des larves et les poussières volatiles. Ce moyen simple et compact permet de récupérer les mues de larves. On utilise par exemple un anneau de Pouyès. Le système permet avantageusement d'assurer une bonne qualité de l'air ambiant.

De manière avantageuse, le bras manipulateur coopère avec un système de localisation et de guidage du bras manipulateur. Ce mode de réalisation permet d'automatiser le bras manipulateur en assurant un fonctionnement sûr, précis, à faible coût.

Selon un mode de réalisation avantageux, les bacs sont empilables et comportent des plots de guidage facilitant leur empilage et pouvant par ailleurs servir de cible pour le système de localisation et guidage.

L'invention prévoit également un procédé de manutention de bacs de production d'insectes par un système de manutention pour effectuer une transition entre deux cycles de production d'insectes ou de larves dans lequel :
une phase de récupération de matières de production provenant d'un cycle antérieur de production de larves ou d'insectes comprend les étapes suivantes:
   i) réception, par un portique d'entrée et sortie, d'une pile de bacs de fin de cycle, chacun des bacs comportant un substrat, une pluralité de larves;
   ii) à l'aide d'un bras manipulateur, dépilage et transport d'un bac de la pile vers un tamiseur-récupérateur;
   iii) déversement du bac acheminé dans le tamiseur-récupérateur à l'aide du bras manipulateur;
   iv) récupération des larves par le tamiseur-récupérateur et acheminement vers un point de récupération des constituants;
   v) récupération des déjections par le tamiseur-récupérateur et acheminement vers; un point de récupération des constituants;
   vi) récupération du substrat résiduel par le tamiseur-récupérateur;
   vii) les étapes ii à vi sont répétées successivement jusqu'à ce que tous les bacs de la pile de fin de cycle soient traités;
une phase de préparation de bacs pour un cycle ultérieur de production de larves comprend les étapes suivantes afin de préparer une pile d'amorce de cycle, dans laquelle, pour chaque bac à préparer :
   viii) réception d'un bac vide;
   ix) ajout d'une dose de matière d'amorce de production dans le bac à l'aide d'un doseur ;
   x) mise en place du bac dans une station d'empilage;
   xi) les étapes viii à x sont répétées pour constituer une pile de bacs d'amorce de cycle par empilage successif des bacs dans une station d'empilage de bacs de bacs d'amorce de cycle.

Ce procédé permet d'effectuer automatiquement de façon simple, efficace et rapide, l'ensemble des opérations de manipulations des bacs de production en fin de cycle de croissance des larves afin notamment de récupérer ces dernières, et en début de cycle de production de larves, afin de charger la matière d'amorce de production requise dans chacun des bacs.

Selon un mode de réalisation avantageux, les phases de récupération de matières de fin de cycle de production et de préparation de bacs avec matières d'amorce de production ultérieure sont imbriquées de façon à ce que une pile de bacs d'amorce de cycle constituée corresponde à une pile de bacs de fin de cycle dépilée.

Selon encore un mode de réalisation avantageux, l'étape d'ajout d'une dose de substrat dans le bac comprends une étape de mesure d'une dose de substrat résiduel et ajout d'un éventuel complément.

Le procédé prévoit avantageusement une étape de récupération des mues de larves et d'éventuelles poussières volatiles par aspiration.

L'invention prévoit enfin un procédé de manutention de bacs de production d'insectes par un système de manutention pour effectuer une transition entre deux cycles de production dans lequel :
une phase de récupération de matières de production provenant d'un cycle antérieur de production d'oeufs comprend les étapes suivantes:
   i) réception, par un portique d'entrée-sortie, d'une pile de bacs de fin de cycle, chacun des bacs comportant un substrat, une pluralité d'oeufs de larves et une pluralité d'insectes reproducteurs ;
   ii) à l'aide d'un bras manipulateur, dépilage et transport d'un bac de la pile vers un tamiseur-récupérateur ;
   iii) déversement du bac acheminé dans le tamiseur-récupérateur à l'aide du bras manipulateur ;
   iv) récupération des insectes pondeurs par le tamiseur-récupérateur et acheminement vers un point de récupération des constituants pour un cycle ultérieur de ponte;
   v) récupération des oeufs et du substrat résiduel par le tamiseur-récupérateur et acheminement vers un point de récupération des constituants pour des cycles ultérieurs de production de larves;
   vi) les étapes ii à v sont répétées successivement jusqu'à ce que tous les bacs de la pile de fin de cycle soient traités;
une phase de préparation de bacs pour un cycle ultérieur de production d'oeufs comprend les étapes suivantes afin de préparer une pile d'amorce de cycle, dans laquelle, pour chaque bac à préparer :
   vii) réception d'un bac vide;
   viii) ajout d'une dose de matière d'amorce de cycle de production ;
   ix) mise en place du bac dans une station d'empilage;
   x) les étapes vii à ix sont répétées pour constituer une pile de bacs d'amorce de cycle par empilage successif des bacs dans station d'empilage de bacs d'amorce de cycle.

Le procédé permet également d'effectuer automatiquement de façon simple, efficace et rapide, l'ensemble des opérations correspondantes pour des cycles de production d'oeufs, afin notamment de récupérer les oeufs en fin de cycle de production, et, en début de cycle, pour charger la matière d'amorce de cycle de production requise dans chacun des bacs. Ainsi, un système unique permet de gérer les deux cycles requis pour obtenir le produit final, à savoir les larves.

Les phases de récupération de matières de fin de cycle de production et de préparation de bacs avec matières d'amorce de production ultérieure sont avantageusement imbriquées de façon à ce que une pile de bacs d'amorce de cycle constituée corresponde à une pile de bacs de fin de cycle dépilée.

De manière avantageuse, le procédé est utilisé pour produire des coléoptères, plus préférentiellement des tenebrio, et encore plus préférentiellement des tenebrio molitor. Ces types d'insectes sont particulièrement bien adaptés à ce type de procédé. Ils comportent par ailleurs de grandes qualités nutritives, dont un taux élevé de protéines.

De manière avantageuse, les insectes produits sont au stade larvaire. En effet, c'est le stade de croissance permettant l'obtention d'un taux élevé de protéines, tout en conservant des cycles de production relativement courts.

### DESCRIPTION DES FIGURES

Tous les détails de réalisation sont donnés dans la description qui suit, complétée par les figures 1 à 10, présentées uniquement à des fins d'exemples non limitatifs, et dans lesquelles:
- la figure 1 est une vue de dessus d'un exemple de réalisation d'un système de manutention de bacs ;
- la figure 2 est montre, en perspective, le tamiseur-récupérateur du système de la figure 1 ;
- la figure 3 est une vue en perspective du tamiseur-récupérateur et de la station de remplissage ;
- la figure 4 est une vue en perspective d'un exemple de préhenseur ;
- la figure 5 montre le préhenseur de la figure 4 avec un bac ;
- la figure 6 est une vue de dessus du préhenseur de la figure 4 ;
- la figure 7 est une représentation schématique d'un exemple de bac vu de dessous ;
- la figure 8 est une vue en perspective du bac de la figure 7;
- la figure 9 présente un exemple d'empilement en mode fonctionnel de deux bacs identiques ;
- la figure 10 présente un exemple d'empilement en mode compact de deux bacs identiques.

### DESCRIPTION DETAILLEE DE L'INVENTION

### DEFINITIONS

Par « *matières de fin de cycle de production »,* on entend des larves, déjections, résidus de mues, restes de nourriture pour le cycle d'élevage de larves ou insectes, et/ou de reproducteurs, oeufs, restes de nourriture et déjections pour le cycle de ponte.

Par « *matières d'amorce de cycle de production »,* on entend de la nourriture avec oeufs, ou nourriture avec insectes reproducteur, selon le cycle.

Par *« substrat »,* on entend une matière constituant le milieu de vie des oeufs, larves, insectes ou reproducteur.

Par « *substrat* résiduel », on entend le substrat présent en fin de cycle de production.

Par « produit final », on entend les larves en fin de croissance ou des coléoptères adultes.

### SYSTEME DE MANUTENTION

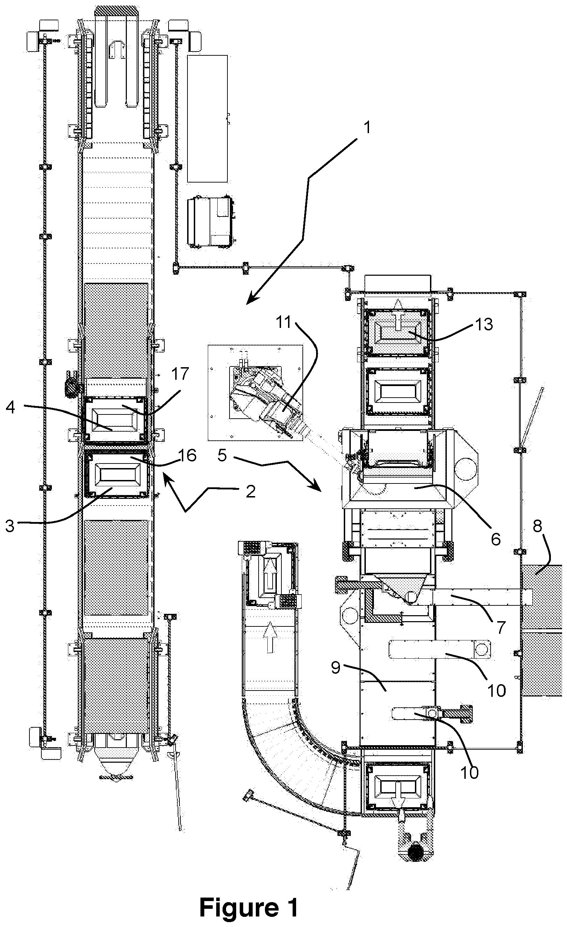

Tel qu'illustré à la figure 1, le système 1 de manutention de bacs 15 de production d'insectes comprend un portique 2 d'entrée et sortie de pile de bacs pour recevoir et mettre en attente une pile 16 de bacs de fin de cycle à traiter et disposer une pile 17 de bacs d'amorce de cycle à préparer. Dans l'exemple illustré, le portique 2 d'entrée et sortie comporte un convoyeur, facilitant l'acheminement des palettes sur lesquelles les piles 16 et 17 de bacs sont posées. Une station 3 de dépilage et une station 4 de dépilage sont prévues pour réaliser les étapes correspondantes. Le portique est accessible depuis l'extérieur du système afin de procéder à l'acheminement des piles de bacs à manipuler.

Le système 1 de manutention comprend également un poste de transition 5, pour effectuer les étapes de transition entre une fin de cycle pour une pile 16 de bacs de fin de cycle et une amorce d'un nouveau cycle en constituant une pile 17 de bacs d'amorce de cycle. En regard des cycles successifs de production à effectuer, ce poste de travail est donc hybride, puisqu'il intervient dans deux cycles successifs de production. Il permet d'effectuer une transition efficace et ergonomique entre deux cycles, en utilisant peu de ressources industrielles et un espace restreint.

Tel qu'illustré, le poste 5 de transition comprend un tamiseur-récupérateur 6 de la matière de fin de cycle, conçu pour séparer les constituants à récupérer de la matière de fin de cycle de production et acheminer ces constituants vers des points 8 de récupération. Le poste 5 de transition comprend également une station 9 de remplissage de bacs comprenant une pluralité de doseurs 10 de constituants de matière d'amorce de cycle de production. Dans l'exemple illustré, un convoyeur assure le flux de bacs entre les différents éléments du poste de transition.

Pour assurer la mise en oeuvre des diverses étapes du procédé de manutention décrit plus loin, un bras manipulateur 11 conçu et agencé pour dépiler séparément chaque bac de la pile 16 de bacs de fin de cycle à traiter, acheminer chaque bac 15 vers le tamiseur-récupérateur 6, verser le contenu de chaque bac issu du cycle de production écoulé dans le tamiseur-récupérateur 6, poser les bacs vides dans une zone de réception de bacs vides, saisir un bac nouvellement rempli dans la station 9 de remplissage, le transporter vers une pile 17 de bacs d'amorce en cours de constitution et effectuer l'empilage des bacs de cette pile.

On prévoit avantageusement un bras manipulateur 11 multiaxes, par exemple cinq ou six axes, permettant d'effectuer avec souplesse les différents mouvements requis pour la mise en oeuvre du procédé, en outre le mouvement d'inclinaison du bac à transvaser, permettant de déverser le contenu du bac dans le tamiseur-récupérateur. Pour assurer une gestion efficace de la dynamique du bras manipulateur, ce dernier coopère avec un système de localisation et de guidage du bras manipulateur (par exemple par vision, ou par bornes ou autre).

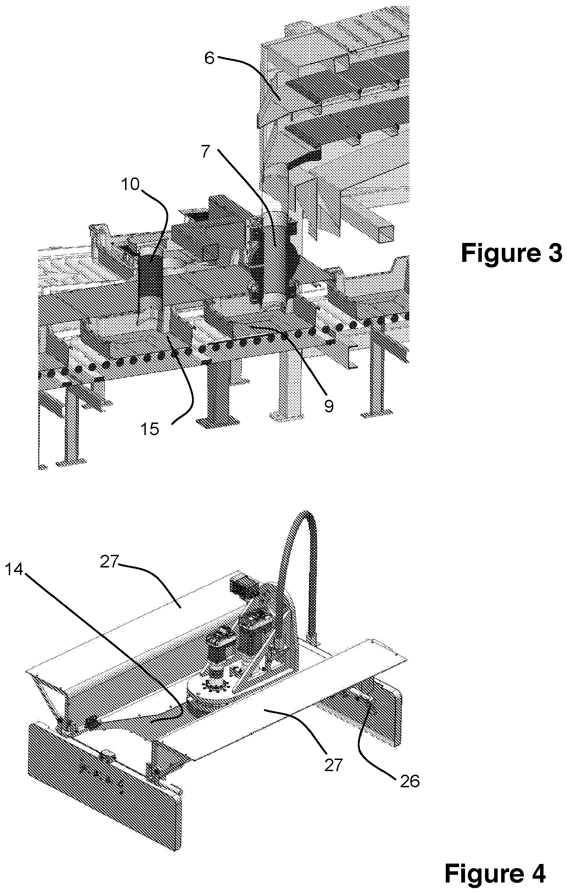

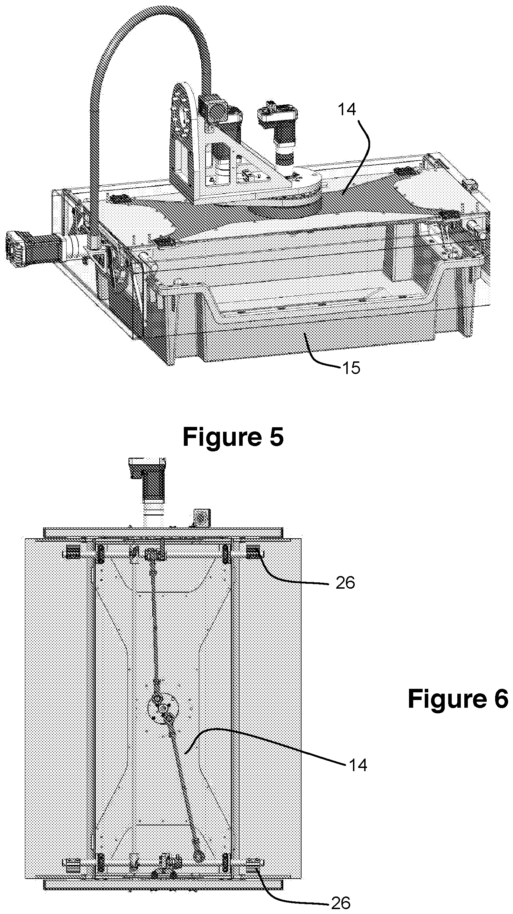

Tel qu'illustré aux figures 4 à 6, un préhenseur 14 est agencé à l'extrémité libre du bras manipulateur 11. Le préhenseur permet d'attraper les bacs, de les manipuler en les soulevant, les inclinant et en les déplaçant. Des panneaux 27 pivotants latéraux montrés en position relevés à la figure 4 et en position fermée à la figure 5, permettent d'enfermer le contenu des bacs pour éviter les pertes de matière lors des manipulations, qui sont avantageusement effectuées à des vitesses importantes. Les figures 4 et 6 illustrent un exemple de réalisation d'un mécanisme d'activation de crochets 26 permettant d'accrocher les bacs par leurs rebords latéraux. Les crochets 26 sont montés pivotant sur une tige rotative au niveau du préhenseur.

Tel qu'illustré à la figure 3, le tamiseur-récupérateur comporte de préférence au moins deux étages. Chaque étage comporte un tamis de granulométrie spécifique, dont les dimensions des orifices sont prévues en fonction des dimensions moyennes des constituants à récupérer. Le tamiseur-récupérateur est de préférence conçu de façon à ce que la récupération des constituants issus d'un cycle de ponte et la récupération des constituants issus d'un cycle d'élevage de larves ou d'insectes puisse être effectuée dans le même tamiseur-récupérateur 6, permettant ainsi de prévoir un unique tamiseur-récupérateur et/ou un unique système de manutention pour le procédé correspondant de mise en oeuvre.

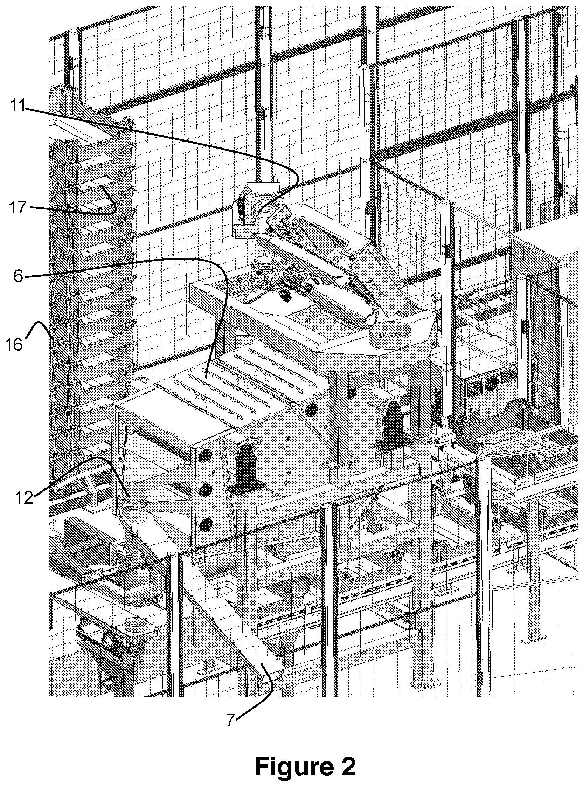

Tel qu'illustré dans l'exemple de la figure 2, le tamiseur-récupérateur comprend un système d'aspiration 12 agencé en sortie de tamisage, et conçu pour aspirer et récupérer les mues des larves issues des multiples mutations des larves au cours de leur croissance.

### PROCEDE

Le système préalablement décrit permet la mise en oeuvre d'un procédé selon deux déclinaisons, soit une première déclinaison pour assurer la transition entre deux cycles de production d'insectes ou larves (ou déclinaison de production du produit final), et une seconde déclinaison pour assurer la transition entre deux cycles de production d'oeufs d'insectes (ou déclinaison de reproduction).

Dans chaque cas, le procédé comporte deux phases, permettant de faire la jonction entre deux cycles successifs de production. On retrouve ainsi une phase de récupération de matières de production provenant d'un cycle antérieur de production de larves ou d'insectes ou d'oeufs et une phase de préparation de bacs pour un cycle ultérieur de production de larves ou d'insectes ou d'oeufs visant à préparer une pile d'amorce de cycle.

### DECLINAISON DE PRODUCTION DU PRODUIT FINAL

Pour produire des insectes ou larves, la première phase du procédé prévoit les étapes suivantes :
i) réception, par un portique d'entrée et sortie, d'une pile de bacs de fin de cycle, chacun des bacs comportant un substrat, une pluralité de larves;
ii) à l'aide d'un bras manipulateur, dépilage et transport d'un bac de la pile vers un tamiseur-récupérateur;
iii) déversement du bac acheminé dans le tamiseur-récupérateur à l'aide du bras manipulateur;
iv) récupération des larves par le tamiseur-récupérateur et acheminement vers un point de récupération des constituants;
v) récupération des déjections par le tamiseur-récupérateur et acheminement vers; un point de récupération des constituants;
vi) récupération du substrat résiduel par le tamiseur-récupérateur;
vii) les étapes ii à vi sont répétées successivement jusqu'à ce que tous les bacs de la pile de fin de cycle soient traités.

Toujours dans le cas de production d'insectes ou larves, la seconde phase comprend les étapes suivantes :
viii) réception d'un bac vide;
ix) ajout d'une dose de matière d'amorce dans le bac à l'aide d'un doseur;
x) mise en place du bac dans une station d'empilage;
xi) les étapes viii à x sont répétées pour constituer une pile de bacs d'amorce de cycle par empilage successif des bacs dans une station d'empilage de bacs de bacs d'amorce de cycle.

Selon un exemple de réalisation, pour bénéficier au mieux des spécificités du système de manutention avec poste de transition entre deux cycles successifs de production, les phases de récupération de matières de fin de cycle de production et de préparation de bacs avec matières d'amorce de production ultérieure sont avantageusement imbriquées de façon à ce qu'une pile de bacs d'amorce de cycle constituée corresponde à une pile de bacs de fin de cycle dépilée.

Le procédé permet une variante dans laquelle on récupère directement un substrat résiduel d'un bac de fin de production, ce substrat étant envoyé dans un bac à remplir pour un cycle suivant. Afin de limiter les pertes, une étape de pesée de la dose de substrat résiduel est alors prévue, soit avant transvasement dans le bac, soit après transvasement dans le bac (en pesant ensemble le bac de masse connue avec le substrat pour en déduire la masse du substrat). En fonction de la masse de substrat résiduel, on prévoit une éventuelle étape d'ajout d'une dose ou masse complémentaire de substrat ou nourriture.

Enfin, la croissance des larves donnant lieu à plusieurs mues, les résidus (ou exuvies) des mues sont avantageusement récupérées. Ces résidus étant en général très légers et sujets à se disperser sous forme de poussière lors des étapes de tamisage et de récupération des constituants, le procédé prévoit avantageusement une étape de récupération des mues de larves, de préférence par aspiration, par exemple à l'aide d'un anneau de Pouyès, prévu dans une des tubulures d'acheminement des constituants vers les points 8 de récupération de constituants.

### DECLINAISON DE REPRODUCTION

Pour produire des oeufs d'insectes, la première phase du procédé prévoit les étapes suivantes :
i) réception, par un portique d'entrée-sortie, d'une pile de bacs de fin de cycle, chacun des bacs comportant un substrat, une pluralité d'oeufs et une pluralité d'insectes reproducteurs;
ii) à l'aide d'un bras manipulateur, dépilage et transport d'un bac de la pile vers un tamiseur-récupérateur;
iii) déversement du bac acheminé dans le tamiseur-récupérateur à l'aide du bras manipulateur ;
iv) récupération des insectes pondeurs par le tamiseur-récupérateur et acheminement vers un point de récupération des constituants pour un cycle ultérieur de ponte;
v) récupération des oeufs et du substrat résiduel par le tamiseur-récupérateur et acheminement vers un point de récupération des constituants pour des cycles ultérieurs de production de larves;
vi) les étapes ii à v sont répétées successivement jusqu'à ce que tous les bacs de la pile de fin de cycle soient traités.

Toujours dans le cas de production d'insectes ou larves, la seconde phase comprend les étapes suivantes :
vii) réception d'un bac vide;
viii) ajout d'une dose de matière d'amorce de cycle de production;
ix) mise en place du bac dans une station d'empilage;
x) les étapes vii à ix sont répétées pour constituer une pile de bacs d'amorce de cycle par empilage successif des bacs dans station d'empilage de bacs d'amorce de cycle.

Ici encore, on prévoit avantageusement que les phases de récupération de matières de fin de cycle de production et de préparation de bacs avec matières d'amorce de production ultérieure sont imbriquées de façon à ce que une pile de bacs d'amorce de cycle constituée corresponde à une pile de bacs de fin de cycle dépilée.

Le système et procédé préalablement décrits sont avantageusement utilisés pour produire des insectes ou larves comestibles, par exemple à de fins de nourriture pour animaux à base d'insectes entiers ou non.

Pour produire de grandes quantités d'insectes ou larves, on met en place des fermes ou usines d'insectes. Une ferme comprend par exemple un ou plusieurs emplacements de stockage pour placer une pluralité de piles de bacs. Un ou plusieurs dispositifs complémentaires peuvent être prévus par exemple pour ajouter des doses d'arrosage lorsque nécessaire ou à des intervalles préétablis.

Le système de manipulation préalablement décrit peut être agencé dans une zone réservée de l'emplacement de stockage, ou dans une pièce ou local avoisinant.

Après leur croissance, les larves ou insectes peuvent subir une ou plusieurs étapes de transformation en produit alimentaire. On prévoit par exemple une phase de déshydratation, afin que le produit alimentaire obtenu soit facile à conserver et à manutentionner.

### BACS DE PRODUCTION

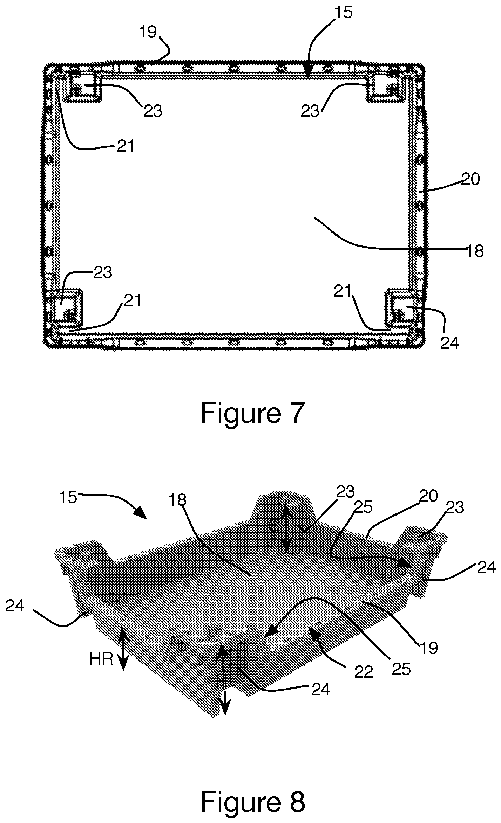

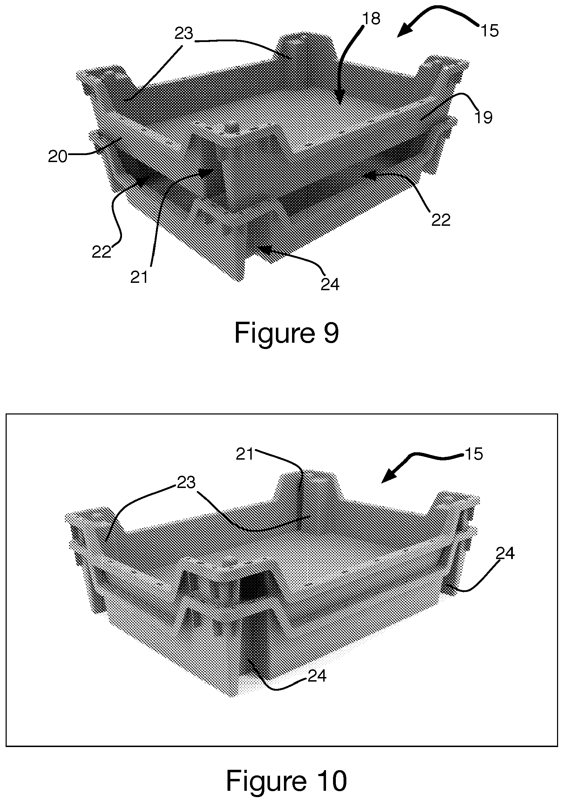

Les figures 7 à 10 sont des représentations schématiques d'un exemple de bac 15 conçu pour la production d'insectes, notamment pour produire de la nourriture pour animaux à base d'insectes. Ces bacs 15 servent à loger les larves en cours de croissance ainsi que les nutriments utilisés pour assurer cette croissance. Au cours de leur croissance, les larves produisent des déjections qui vont également être contenus dans les bacs. En pratique, pour utiliser un espace le plus restreint possible, les bacs sont empilés les uns sur les autres, tel que montré dans l'exemple de la figure 9, pour former des piles de bacs pouvant comprendre cinq à dix bacs ou quinze bacs, voire plus.

Chacun des bacs comprend un fond 18 plat rectangulaire, entouré par deux parois latérales longues 19 opposées et deux parois latérales courtes 20 opposées, les parois étant de hauteur H, cette hauteur étant au moins présente au niveau des jonctions des parois 19 et 20. En variante, les bacs sont carrés, avec des parois de même longueur.

Pour assurer une bonne oxygénation des larves et pour faciliter la ventilation, les bacs sont prévus avec des fenêtres latérales 22. Tel que montré dans l'exemple de la figure 8, les fenêtres latérales sont formées par un double décrochement 25 des parois longues et/ou des parois courtes. On obtient ainsi une zone médiane de hauteur HR réduite formant une fenêtre latérale 22 d'aération. Cette fenêtre latérale doit par ailleurs être libre lorsque les bacs sont empilés les uns dans les autres en position fonctionnelle.

Pour permettre d'empiler les bacs les uns par dessus les autres, chaque bac comporte des éléments de support conçus pour permettre de positionner deux bacs identiques l'un dans l'autre en laissant les fenêtres latérales 22 au moins partiellement libres, à des fins d'aération.

Tel qu'illustré aux figures 7 et 8, les éléments de support sont réalisés sous la forme de colonnes 23 ou tiges ou piliers internes au bac, de préférence formées par un renfoncement 24 vers l'intérieur du bac d'une portion des parois latérales du bac. La hauteur C des colonnes est de préférence au moins égale à la hauteur HR réduite des fenêtres latérales, et préférentiellement légèrement inférieure à la hauteur H des parois.

En plus de faciliter la fabrication, ce mode de mise en oeuvre permet de libérer l'espace interne des colonnes, de façon à permettre leur empilement en position basse, tel que montré dans l'exemple de la figure 10. En position basse, les fenêtres latérales 22 sont de préférence masquées par la paroi partiellement imbriquée du bac supérieur. Les éléments permettant de choisir une position d'empilement fonctionnelle (haute avec fenêtre latérale dégagée) ou basse (avec fenêtre latérale non dégagée) sont décrits dans ce qui suit.

Tel que visible plus particulièrement aux figures 9 et 10, un bac comporte deux colonnes 23 opposées formées par un renfoncement 24 des parois courtes 20 opposées, et deux colonnes formées par un renfoncement 24 d'une même paroi longue 19, au voisinage des extrémités de cette paroi. Cet agencement asymétrique, couplé à la forme inclinée des parois des colonnes de sorte que le périmètre décroisse progressivement avec la hauteur de la colonne, ainsi que la caractéristique selon laquelle les colonnes 23 sont agencées de façon à former une fente 21 allongée s'étendant entre la colonne et la paroi adjacente à la paroi dans laquelle la colonne est formée, permettent de réaliser des empilages selon les deux modes préalablement décrits.

Tel que décrit et illustré, l'architecture asymétrique du bac, en particulier des colonnes 23, permettent qu'un bac identique posé sur les colonnes dudit bac soit porté en position haute lorsque les colonnes de paroi longue d'un bac supérieur sont posées sur les colonnes de paroi courte d'un bac inférieur, et porté en position basse lorsque les colonnes de paroi longue d'un bac supérieur sont posées sur les colonnes de paroi longue d'un bac inférieur. Autrement dit, la position fonctionnelle est obtenue lorsque deux bacs identiques sont empilés avec un alignement symétrique des colonnes des deux bacs, et la position de rangement compact est obtenue lorsque deux bacs identiques sont emboîtés avec un alignement asymétrique, ou inverse, des colonnes des deux bacs.

En position haute fonctionnelle, les bacs étant gerbés, la ventilation est assurée par les fenêtres latérales 22. Lorsque les bacs ne sont pas en cours d'utilisation, il est utile de pouvoir les ranger en position basse par emboîtage, afin de minimiser le volume de stockage ou de transport requis.

**Numéros de référence employés sur les figures**

| | |
|---|---|
| 1 | Système de manutention de bacs de production d'insectes |
| 2 | Portique d'entrée et sortie de piles de bacs |
| 3 | Station de dépilage de bacs de fin de cycle |
| 4 | Station d'empilage de bacs d'amorce de cycle |
| 5 | Poste de transition (fin de cycle et amorce d'un nouveau cycle) |
| 6 | Tamiseur-récupérateur pour matière de fin de cycle |
| 7 | Conduits de récupération de constituants |
| 8 | Conduits de récupération de constituants |
| 9 | Station de remplissage de bacs pour matière d'amorce de cycle |
| 10 | Doseurs de constituants de matière d'amorce de cycle |
| 11 | Bras manipulateur unique |
| 12 | Système d'aspiration pour mues de larves |
| 13 | Zone de réception de bacs vides |
| 14 | Préhenseur |
| 15 | Bacs |
| 16 | Pile de bacs de fin de cycle |
| 17 | Pile de bacs d'amorce de cycle |
| 18 | Fond de bac |
| 19 | Paroi latérale longue |
| 20 | Paroi latérale courte |
| 21 | Fente allongée |
| 22 | Fenêtre latérale d'aération |
| 23 | Colonne interne (de support) |
| 24 | Renfoncement de paroi |
| 25 | Double décrochage |
| 26 | Crochets |
| 27 | Panneaux pivotants |

## Revendications

1. Système (1) de manutention de bacs (15) de production d'insectes, pour effectuer la transition entre la fin d'un cycle de production d'une pile de bacs (16) de fin de cycle avec transvasement de la matière de production de chacun des bacs de la pile de fin de cycle, la séparation et récupération des constituants de la matière de production, et l'amorce d'un nouveau cycle de production avec remplissage de bacs de matière d'amorce de production et constitution d'une pile de bacs (17) d'amorce de cycle, ledit système comprenant :
i) un portique (2) d'entrée et sortie de piles de bacs pour recevoir et mettre en attente une pile de bacs (16) de fin de cycle à traiter et disposer une pile de bacs (17) d'amorce de cycle;
ii) un poste de transition (5) entre une fin de cycle pour une pile de bacs (16) de fin de cycle et une amorce d'un nouveau cycle pour constituer une pile de bacs (17) d'amorce de cycle, le poste de transition (5) comprenant :
- un tamiseur-récupérateur (6) pour matière de fin de cycle pour séparer les constituants à récupérer de la matière de fin de cycle de production et acheminer ces constituants vers des points (8) de récupération;
- une station (9) de remplissage de bacs comprenant une pluralité de doseurs (10) de constituants de matière d'amorce de cycle de production;
iii) un bras manipulateur (11) pour dépiler séparément chaque bac de la pile (16) de bacs (15) de fin de cycle à traiter, acheminer chaque bac vers le tamiseur-récupérateur (6), verser le contenu de chaque bac dans le tamiseur-récupérateur, poser les bacs vides dans une zone de réception de bacs vides, saisir un bac nouvellement rempli dans la station (9) de remplissage, le transporter vers une pile de bacs (17) d'amorce en cours de constitution et effectuer l'empilage des bacs de cette pile.

2. Système de manutention selon la revendication 1, dans lequel le tamiseur-récupérateur (6) comporte au moins deux étages.

3. Système de manutention selon l'une quelconque des revendications 1 ou 2, dans lequel le tamiseur-récupérateur (6) est configuré de façon à ce que la récupération des constituants pour un cycle de ponte et la récupération des constituants pour un cycle d'élevage de larves ou d'insectes soit effectuée dans le même tamiseur-récupérateur (6).

4. Système de manutention selon l'une quelconque des revendications 1 à 3, dans lequel le tamiseur-récupérateur (6) comprend un système d'aspiration(12) agencé pour récupérer les mues des larves.

5. Système de manutention selon l'une quelconque des revendications 1 à 4, dans lequel le bras manipulateur (11) coopère avec un système de localisation et de guidage du bras manipulateur.

6. Procédé de manutention de bacs (15) de production d'insectes par un système (1) de manutention pour effectuer une transition entre deux cycles de production d'insectes ou de larves dans lequel :
une phase de récupération de matières de production provenant d'un cycle antérieur de production de larves ou d'insectes comprend les étapes suivantes:
i) réception, par un portique (2) d'entrée et sortie, d'une pile (16) de bacs de fin de cycle, chacun des bacs comportant un substrat, et une pluralité de larves;
ii) à l'aide d'un bras (11) manipulateur, dépilage et transport d'un bac de la pile de bacs de fin de cycle vers un tamiseur-récupérateur (6);
iii) déversement du bac acheminé dans le tamiseur-récupérateur à l'aide du bras manipulateur;
iv) récupération des larves par le tamiseur-récupérateur et acheminement vers un point (8) de récupération des constituants;
v) récupération des déjections par le tamiseur-récupérateur et acheminement vers un point (8) de récupération des constituants;
vi) récupération du substrat résiduel par le tamiseur-récupérateur;
vii) répétition successive des étapes ii à vi jusqu'à ce que tous les bacs de la pile (16) de fin de cycle soient traités; une phase de préparation de bacs pour un cycle ultérieur de production de larves comprend, pour chaque bac à préparer, les étapes suivantes afin de préparer une pile (17) d'amorce de cycle:
viii) réception d'un bac vide;
ix) ajout d'une dose d'amorce de cycle dans le bac à l'aide d'un doseur (10);
x) mise en place du bac dans une station (4) d'empilage;
xi) répétition des étapes viii à x pour constituer une pile (17) de bacs d'amorce de cycle par empilage successif des bacs dans la station (4) d'empilage de bacs de bacs d'amorce de cycle.

7. Procédé de manutention de bacs selon la revendication 6, dans lequel les phases de récupération de matières de fin de cycle de production et de préparation de bacs avec matières d'amorce de production ultérieure sont imbriquées de façon à ce que une pile (17) de bacs d'amorce de cycle constituée corresponde à une pile (16) de bacs de fin de cycle dépilée.

8. Procédé de manutention de bacs selon l'une quelconque des revendications 6 ou 7, comprenant une étape de récupération des mues de larves par aspiration.

9. Procédé de manutention de bacs (15) de production d'insectes par un système (1) de manutention pour effectuer une transition entre deux cycles de production d'oeufs dans lequel :
une phase de récupération de matières de production provenant d'un cycle antérieur de production d'oeufs comprend les étapes suivantes:
i) réception, par un portique (2) d'entrée-sortie, d'une pile (16) de bacs de fin de cycle, chacun des bacs (15) comportant un substrat, une pluralité d'oeufs et une pluralité d'insectes reproducteurs;
ii) à l'aide d'un bras (11) manipulateur, dépilage et transport d'un bac de la pile (16) de bacs de fin de cycle vers un tamiseur-récupérateur (6);
iii) déversement du bac (15) acheminé dans le tamiseur-récupérateur à l'aide du bras manipulateur (11) ;
iv) récupération des insectes pondeurs par le tamiseur-récupérateur et acheminement vers un point (8) de récupération des constituants pour un cycle ultérieur de ponte;
v) récupération des oeufs et du substrat résiduel par le tamiseur-récupérateur et acheminement vers un point (8) de récupération des constituants pour des cycles ultérieurs de production de larves;
vi) répétition successive des étapes ii à jusqu'à ce que tous les bacs de la pile (16) de fin de cycle soient traités; une phase de préparation de bacs pour un cycle ultérieur de production d'oeufs comprend, pour chaque bac à préparer, les étapes suivantes afin de préparer une pile (17) d'amorce de cycle:
vii) réception d'un bac (15) vide;
viii) ajout d'une dose de matière d'amorce de cycle de production dans le bac ;
ix) mise en place du bac dans une station (4) d'empilage;
x) répétition des étapes vii à ix pour constituer une pile (17) de bacs d'amorce de cycle par empilage successif des bacs dans la station (4) d'empilage de bacs d'amorce de cycle

10. Procédé de manutention de bacs selon la revendication 9, dans lequel les phases de récupération de matières de fin de cycle de production et de préparation de bacs avec matières d'amorce de production ultérieure sont imbriquées de façon à ce que une pile (17) de bacs d'amorce de cycle constituée corresponde à une pile (16) de bacs de fin de cycle dépilée.

## Patentansprüche

1. System (1) zur Förderung von Behältern (15) zur Produktion von Insekten, um den Übergang zwischen dem Ende eines Produktionszyklus eines Zyklusendestapels (16) von Behältern mit Umfüllen des Produktionsstoffs jedes der Behälter des Zyklusendestapels, die Trennung und Rückgewinnung der Bestandteile des Produktionsstoffs und das Anfangen eines neuen Produktionszyklus mit Füllen von Behältern mit Produktionsanfangsstoff und Bilden eines Zyklusanfangsstapels (17) von Behältern durchzuführen, wobei das System Folgendes umfasst:
i) ein Eingangs- und Ausgangsportal (2) von Behälterstapeln zum Empfangen und Bereithalten eines Zyklusendestapels (16) von Behältern, die zu verarbeiten sind, und Anordnen eines Zyklusanfangsstapels (17) von Behältern; ii) einen Übergangsposten (5) zwischen einem Zyklusende für den Zyklusendestapel (16) von Behältern und einem Anfang eines neuen Zyklus, um einen Zyklusanfangsstapel (17) von Behältern zu bilden, wobei der Übergangsposten (5) Folgendes umfasst:
- eine Sieb-Rückgewinnungsvorrichtung (6) für Zyklusendestoff, um die Bestandteile abzutrennen, die von dem Stoff am Produktionszyklusende zurückgewonnen werden sollen, und diese Bestandteile zu Rückgewinnungsstellen (8) zu befördern;
- eine Füllstation (9) von Behältern, die eine Vielzahl von Dosierern (10) von Bestandteilen von Produktionszyklus-Anfangsstoff umfasst;
iii) einen Manipulatorarm (11), um jeden Behälter des Zyklusendestapels (16) von Behältern (15), der verarbeitet werden soll, separat zu entstapeln, jeden Behälter zu der Sieb-Rückgewinnungsvorrichtung (6) zu befördern, den Inhalt jedes Behälters in die Sieb-Rückgewinnungsvorrichtung zu schütten, die leeren Behälter in einer Empfangszone für leere Behälter abzustellen, einen neu gefüllten Behälter in der Füllstation (9) zu erfassen, ihn zu einem Zyklusanfangsstapel (17) von Behältern, der sich in Bildung befindet, zu transportieren, und das Stapeln der Behälter dieses Stapels durchzuführen.

2. Fördersystem nach Anspruch 1, wobei die Sieb-Rückgewinnungsvorrichtung (6) mindestens zwei Etagen aufweist.

3. Fördersystem nach einem der Ansprüche 1 oder 2, wobei die Sieb-Rückgewinnungsvorrichtung (6) derart konfiguriert ist, dass die Rückgewinnung der Bestandteile für einen Legezyklus und die Rückgewinnung der Bestandteile für einen Larven- oder Insektenaufzuchtzyklus in derselben Sieb-Rückgewinnungsvorrichtung (6) erfolgen.

4. Fördersystem nach einem der Ansprüche 1 bis 3, wobei die Sieb-Rückgewinnungsvorrichtung (6) ein Ansaugsystem (12) umfasst, das eingerichtet ist, um die Abhäutungen der Larven zurückzugewinnen.

5. Fördersystem nach einem der Ansprüche 1 bis 4, wobei der Manipulatorarm (11) mit einem Lokalisierungs- und Führungssystem des Manipulatorarms zusammenwirkt.

6. Verfahren zum Fördern von Behältern (15) zur Produktion von Insekten durch ein Fördersystem (1), um einen Übergang zwischen zwei Insekten- oder Larvenproduktionszyklen durchzuführen, wobei:
eine Rückgewinnungsphase von Produktionsstoffen, die aus einem früheren Larven- oder Insektenproduktionszyklus stammen, die folgenden Schritte umfasst:
i) Empfang, durch ein Eingangs- und Ausgangsportal (2), eines Zyklusendestapels (16) von Behältern, wobei jeder der Behälter ein Substrat und eine Vielzahl von Larven umfasst,
ii) mit Hilfe eines Manipulatorarms (11), Entstapeln und Transport eines Behälters des Zyklusendestapels zu einer Sieb-Rückgewinnungsvorrichtung (6);
iii) Ausschütten des beförderten Behälters in die Sieb-Rückgewinnungsvorrichtung mit Hilfe des Manipulatorarms;
iv) Rückgewinnung der Larven durch die Sieb-Rückgewinnungsvorrichtung und Befördern zu einer Rückgewinnungsstelle (8) der Bestandteile;
v) Rückgewinnung der Ausscheidungen durch die Sieb-Rückgewinnungsvorrichtung und Befördern zu einer Rückgewinnungsstelle (8) der Bestandteile;
vi) Rückgewinnung des restlichen Substrats durch die Sieb-Rückgewinnungsvorrichtung;
vii) aufeinanderfolgende Wiederholung der Schritte ii) bis vi), bis alle Behälter des Zuklusendestapels (16) verarbeitet sind;
eine Vorbereitungsphase von Behältern für einen späteren Larvenproduktionszyklus für jeden vorzubereitenden Behälter die folgenden Schritte umfasst, um einen Zyklusanfangsstapel (17) vorzubereiten:
viii) Empfangen eines leeren Behälters;
ix) Hinzufügung einer Zyklusanfangsdosis in den Behälter mit Hilfe eines Dosierers (10);
x) Aufstellen des Behälters in einer Stapelungsstation (4);
xi) Wiederholung der Schritte viii) bis x), um einen Zyklusanfangsstapel (17) von Behältern durch aufeinander folgendes Stapeln der Behälter in der Behälterstapelungsstation (4) von Zyklusanfangsbehältern zu bilden.

7. Förderverfahren von Behältern nach Anspruch 6, wobei die Rückgewinnungsphasen von Produktionszyklusendestoffen und die Vorbereitung von Behältern mit Anfangsstoffen zur späteren Produktion derart verschachtelt sind, dass ein gebildeter Zyklusanfangsstapel (17) von Behältern einem Zyklusendestapel (16) von Behältern entspricht.

8. Förderverfahren von Behältern nach einem der Ansprüche 6 oder 7, das einen Rückgewinnungsschritt der Larvenabhäutungen durch Ansaugung umfasst.

9. Förderverfahren von Behältern (15) zur Produktion von Insekten durch ein Fördersystem (1), um einen Übergang zwischen zwei Eiproduktionszyklen durchzuführen, wobei:
eine Rückgewinnungsphase von Produktionsstoffen, die aus einem früheren Eiproduktionszyklus stammen, die folgenden Schritte umfasst:
i) Empfang durch ein Eingangs- Ausgangsportal (2) eines Zyklusendestapels (16) von Behältern, wobei jeder der Behälter (15) ein Substrat, eine Vielzahl von Eiern und eine Vielzahl von Fortpflanzungsinsekten umfasst;
ii) mit Hilfe eines Manipulatorarms (11) Entstapeln und Transport eines Behälters des Zyklusendestapels (16) von Behältern zu einer Sieb-Rückgewinnungsvorrichtung (6);
iii) Ausschütten des beförderten Behälters (15) in die Sieb-Rückgewinnungsvorrichtung mit Hilfe des Manipulatorarms (11);
iv) Rückgewinnung der Legeinsekten durch die Sieb-Rückgewinnungsvorrichtung, und Beförderung zu einer Rückgewinnungsstelle (8) der Bestandteile für einen späteren Legezyklus;
v) Rückgewinnung der Eier und des restlichen Substrats durch die Sieb-Rückgewinnungsvorrichtung und Beförderung zu einer Rückgewinnungsstelle (8) der Bestandteile für spätere Larvenproduktionszyklen;
vi) aufeinanderfolgende Wiederholung der Schritte ii) bis v), bis alle Behälter des Zyklusendestapels (16) verarbeitet sind;
eine Vorbereitungsphase von Behältern für einen späteren Eiproduktionszyklus für jeden vorzubereitenden Behälter die folgenden Schritte umfasst, um einen Zyklusanfangsstapel (17) vorzubereiten:
vii) Empfang eines leeren Behälters (15);
viii) Hinzufügung einer Dosis von Produktionszyklus-Anfangsstoff in den Behälter;
ix) Aufstellen des Behälters in einer Stapelungsstation (4);
x) Wiederholung der Schritte vii) bis ix), um einen Zyklusanfangsstapel (17) von Behältern durch aufeinanderfolgendes Stapeln der Behälter in der Stapelungsstation (4) von Zyklusanfangsbehältern zu bilden.

10. Förderverfahren von Behältern nach Anspruch 9, wobei die Rückgewinnungsphasen von Produktionszyklusendestoffen und die Vorbereitung von Behältern mit Anfangsstoffen zur späteren Produktion derart verschachtelt sind, dass ein gebildeter Zyklusanfangsstapel (17) von Behältern einem Zyklusendestapel (16) von Behältern entspricht.

## Claims

1. System (1) for handling insect production trays (15), to carry out the transition between the end of a production cycle of a stack of trays (16) with transfer of the production material of each of the trays of the end of cycle stack, the separation and collection of the constituents of the production material, and the initiation of a new production cycle with filling of the trays with production initiation material and making a new stack of cycle initiation trays (17), the said system comprising:
i) an entry and exit gate (2) for stacks of trays to receive and hold a stack of end of cycle trays (16) to process and to make available a stack of cycle initiation trays (17);
ii) a transition station (5) between an end of cycle for a stack of end of cycle trays (16) and initiation of a new cycle to make a stack of cycle initiation trays (17), the transition station (5) comprising:
- a sieve-collector (6) for end of cycle material to separate the constituents to collect at end of production cycle and to convey the constituents to the collection points (8);
- a tray filling station (9) comprising a plurality of dispensers (10) of constituents of production cycle initiation material;
iii) a manipulator arm (11) to separately unstack each tray from the stack (16) of end of cycle trays (15) to be processed, convey each tray to the sieve-collector (6), empty the contents of each tray into the sieve-collector, place the empty trays in an empty tray reception zone, take a newly filled tray into the filling station (9), send it to a stack of initiation trays (17) being assembled and stack the trays of this stack.

2. Handling system according to claim 1, wherein the sieve-collector (6) comprises at least two levels.

3. Handling system according to any one of claims 1 or 2, wherein the sieve-collector (6) is configured such that the collection of constituents for a laying cycle and the collection of constituents for a larva or insect rearing cycle is carried out in the same sieve-collector (6).

4. Handling system according to any one of claims 1 to 3, wherein the sieve-collector (6) comprises a suction system (12) arranged to collect the larva moults.

5. Handling system according to any one of claims 1 to 4, wherein the manipulator arm (11) cooperates with a location and guidance system of the manipulator arm.

6. Process for handling insect production trays (15) by a handling system (1) to carry out a transition between two insect or larva production cycles in which:
a phase of collecting production material from a previous larva or insect production cycle comprises the following steps:
i) receive by an entry and exit gate (2) a stack (16) of end of cycle trays, each of the trays comprising a substrate and a plurality of larvae;
ii) using a manipulator arm (11), unstack and transport a stack of end of cycle trays to a sieve-collector (6);
iii) empty the conveyed tray into the sieve-collector using the manipulator arm;
iv) collect larvae with the sieve-collector and take them to a constituent collection point (8) ;
v) collect the dejections from the sieve-collector and send them to a constituent collection point (8);
vi) collect the residual substrate with the sieve-collector;
vii) successively repeat steps ii to vi until all the end of cycle trays in the stack (16) have been processed;
a tray preparation phase for a later larva production cycle comprises, for each tray to prepare,
the following steps to prepare a cycle initiation stack (17):
viii) receive an empty tray;
ix) add a dose of cycle initiator to the tray using a dispenser (10);
x) place the tray in a stacking station (4);
xi) repeat steps viii to x to make a stack (17) of cycle initiation trays by successively stacking trays in the tray stacking station (4) of cycle initiation trays.

7. Tray handling process according to claim 6, wherein the phases of collecting end of production cycle material and preparing trays with initiation material for later production are interconnected such that a stack (17) of composed cycle initiation trays corresponds to a stack (16) of unstacked end of cycle trays.

8. Tray handling process according to any one of claims 6 or 7, comprising a step for collecting larva moults by suction.

9. Handling process for insect production trays (15) by a handling system (1) to carry out the transition between two egg production cycles in which:
a phase of collecting production material from a previous egg production cycle comprises the following steps:
i) receive by an entry-exit gate (2) a stack (16) of end of cycle trays, each of the trays (15) comprising a substrate, a plurality of eggs and a plurality of reproducing insects;
ii) using a manipulator arm (11), unstack and transport a tray from the stack (16) of end of cycle trays to a sieve-collector (6);
iii) empty the conveyed tray (15) into the sieve-collector using the manipulator arm (11);
iv) collect the laying insects with the sieve-collector and send them to a constituent collection point (8) for a later laying cycle;
v) collect the eggs and residual substrate with the sieve-collector and send them to a constituent collection point (8) for later larva production cycles;
vi) successively repeat steps ii to v until all the trays in the end of cycle stack (16) have been processed;
a phase of preparing trays for a later egg production cycle comprises, for each tray to be prepared,
the following steps to prepare a cycle initiation stack (17):
vii) receive an empty tray (15);
viii) add a dose of production cycle initiation material to the tray;
ix) place the tray in a stacking station (4);
x) repeat steps vii to ix to make a stack (17) of cycle initiation trays by successive stacking of trays in the cycle initiation tray stacking station (4)

10. Tray handling process according to claim 9, wherein the phases of collecting end of production cycle material and preparing trays with initiation material for later production are interconnected such that a stack (17) of composed cycle initiation trays corresponds to a stack (16) of end of cycle trays unstacked.
